# EUROPEAN PATENT SPECIFICATION

(11) **EP 1 436 384 B1**
(45) Date of publication and mention of the grant of the patent: **17.06.2009**
(21) Application number: 02782829.2
(22) Date of filing: 04.10.2002
(51) Int. Cl.: C12N 9/12

(54) **MUTATED ABL KINASE DOMAINS**
MUTIERTE ABL-KINASE-DOMÄNEN
DOMAINES DE L'ABL KINASE MUTES

(30) Priority: 05.10.2001 US 327389 P; 12.10.2001 US 328740 P; 11.01.2002 US 347351 P
(43) Date of publication of application: 14.07.2004
(60) Divisional application: 08166771.9
(73) Proprietor: NOVARTIS AG, 4056 Basel (CH); Université Victor Segalen Bordeaux 2, 33076 Bordeaux Cedex (FR); Technische Universität München, 80333 München (DE); Oregon Health & Science University, Portland, OR 97201-4753 (US); Universität Heidelberg, 69117 Heidelberg (DE); CHRU DE LILLE, centre Hospitalier Regional Universitaire de Lille, 59037 Lille Cedex (FR); MEDVET SCIENCE PTY. LTD., Thebarton, S.A. 5031 (AU)
(72) Inventor: BARTHE, Christophe, F-33127 Martignas Sur Jalles (FR); BRANFORD, Susan, Flagstaff Hill, S.A. 5159 (AU); CORBIN, Amie, Portland, OR 97219 (US); DRUKER, Brian, Jay, Portland, OR 97239 (US); DUYSTER, Justus, 81739 München (DE); HOCHHAUS, Andreas, 68163 Mannheim (DE); HUGHES, Timothy, Kensington Gardens, S.A. 5068 (AU); KREIL, Sebastian, 68165 Mannheim (DE); LEGUAY, Thibaut, F-33000 Bordeaux (FR); MAHON, François-Xavier, F-33000 Bordeaux (FR); MARIT, Gérald, F-33200 Bordeaux (FR); MÜLLER, Martin, 69120 Heidelberg (DE); PESCHEL, Christian, 81739 München (DE); PREUDHOMME, Claude, F-62150 Houdain (FR); ROCHE LESTIENNE, Catherine, F-59800 Lille (FR); RUDZKI, Zbigniew, Rostrevor, S.A. 5073 (AU)
(74) Representative: de Weerd, Petrus G.W.
(86) International application number: PCT/EP2002/011144
(87) International publication number: WO 2003/031608

(56) References cited:
- WO-A-02/102976
- GORRE MERCEDES E ET AL: "Clinical resistance to STI-571 cancer therapy caused by BCR-ABL gene mutation or amplification." SCIENCE (WASHINGTON D C), vol. 293, no. 5531, 2001, pages 876-880, XP002229884 ISSN: 0036-8075
- BARTHE C ET AL: "Roots of clinical resistance to STI-571 cancer therapy." SCIENCE. UNITED STATES 21 SEP 2001, vol. 293, no. 5538, 21 September 2001 (2001-09-21), page 2163 XP002229885 ISSN: 0036-8075
- DATABASE EMBL [Online] 1 November 1995 (1995-11-01) OTTILIE ET AL.: "Tyrosine-protein kinase isoform SRK1" retrieved from EBI Database accession no. X61601; P42686 XP002229888
- SCHINDLER THOMAS ET AL: "Structural mechanism for STI-571 inhibition of Abelson tyrosine kinase." SCIENCE (WASHINGTON D C), vol. 289, no. 5486, 2000, pages 1938-1942, XP002229886 ISSN: 0036-8075 cited in the application
- TSAO A S ET AL: "STI-571 in chronic myelogenous leukaemia." BRITISH JOURNAL OF HAEMATOLOGY, vol. 119, no. 1, October 2002 (2002-10), pages 15-24, XP002229887 October, 2002 ISSN: 0007-1048

## Description

### Field of the Invention:

This invention relates to isolated polypeptides which comprise a functional kinase domain comprising the amino acid sequence of the native human Abl kinase domain or an essentially similar sequence thereof in which Met244, is replaced by another amino acid, said mutated functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof, to the use of such polypeptides to screen for compounds which inhibit the tyrosine kinase activity of such polypeptides, to nucleic acid molecules encoding such polypeptides, to recombinant vectors and host cells comprising such nucleic acid molecules and to the use of such nucleic acid molecules in the production of such polypeptides for use in screening for compounds which inhibit the tyrosine kinase activity of such polypeptides.

### Background of the Invention:

Bcr-Abl, a constitutively activated tyrosine kinase resulting from the formation of the Philadelphia chromosome [Nowell P.C..and Hungerford D.A., Science 132, 1497 (1960)] by reciprocal translocation between the long arms of chromosomes 9 and 22 [Rowley J.D., Nature 243, 290-293 (1973)], has been established as the characteristic molecular abnormality present in virtually all cases of chronic myeloid leukemia (CML) and up to 20 percent of adult acute lymphoblastic leukemia (ALL) [Faderl S. et al., N Engl J Med 341, 164-172 (1999); Sawyers C.L., N Engl J Med 340, 1330-1340 (1999)]. Bcr-Abl is sufficient to cause CML in mice [Daley G.Q. et al., Science 247, 824-830 (1990)] and its transforming capacity is absolutely dependent on tyrosine kinase activity [Lugo T.G. et al., Science 247, 1079 (1990)]. The compound N-[4-methyl-3-(4-pyridin-3-yl-pyrimidin-2-ylamino)-phenyl]-4-(4 methyl-piperazin-1-ylmethyl)-benzamide (hereinafter also referred to as "STI571"; STI571 is described in EP 0 564 409 and, in the form of the methane sulfonate salt, in WO 99/03854), a competitive inhibitor at the ATP-binding site of Bcr-Abl, as well as of the receptor for platelet-derived growth factor, and c-kit tyrosine kinase [Lugo T.G. et al., Science 247, 1079 (1990)], has been shown to be capable of very rapidly reversing the clinical and hematological abnormalities of CML in chronic phase and in blast crisis as well as of Ph chromosome-positive (Ph+) acute lymphoblastic leukemia (Ph+ ALL) [Druker B.J. et al., N Engl J Med 344, 1031-1037 (2001); Druker B.J. et al., N Engl J Med 344, 1038-1042 (2001)]. Whereas almost all chronic phase CML patients durably respond, remissions in CML blast crisis and Ph+ ALL are transient, and most patients relapse after several months, despite continued therapy with STI571 [Druker B. J. et al., N Engl J Med 344, 1038-1042 (2001)]. The mechanism of resistance to STI571 is subject of intense research.
It was now surprisingly found that mutations present in the kinase domain of the Bcr-Abl gene of patients suffering from CML or Ph+ ALL account for the biological resistance of these patients towards STI571 treatment in that said mutations lead to resistance of the Bcr-Abl tyrosine kinase towards inhibition by STI571.
These findings are extremely valuable in e.g. finding new compounds or combinations of compounds which are capable to overcome resistance towards treatment with STI571. Moreover, knowledge of such mutations is also very useful in the diagnosis of Ph+ leukemias in that it allows e.g. the detection of drug-resistant clones before clinical relapse of the patient.

### Definitions:

Within the context of this disclosure the following expressions, terms and abbreviations have the meanings as defined below:

In the expression "a functional kinase domain", the term "functional" indicates that the respective kinase domain possesses tyrosine kinase activity. Preferably, the kinase activity of such a functional kinase domain is in the range of that of the native human Abl kinase domain.

In the expression "a functional kinase domain being resistant to inhibition of its tyrosine kinase activity by STI571 or a salt thereof", the term "resistant" means that STI571 inhibits the respective functional kinase domain with an IC₅₀ that is higher than that of the native human Abl kinase domain, i.e. higher than about 0.025 µM, preferably higher than about 0.15 µM, more preferably higher than about 0.25 µM, most preferably higher than about 5 µM.

In the expression "amino acid sequence of the native human Abl kinase domain or an essentially similar sequence thereof", the part "or an essentially similar sequence thereof" refers to the amino acid sequence of the native human Abl kinase domain containing mutations, including amino acid exchanges, amino acid deletions and/or amino acid additions, that are not essential for the functionality of the kinase and its resistance to inhibition by STI571 or a salt thereof within the meaning of the term "functional" and "resistant" as defined hereinabove.

The expression "replaced by another amino acid" refers to the replacement of a certain natural amino acid by another natural amino acid.

The names of the amino acids are either written out or the one letter or three letter codes are used. Mutations are referred to by accepted nomenclature, e.g. "Ala380Thr" or "380 Ala→Thr" both indicating that alanine at position 380 is replaced by threonine.

SEQ ID NO:1 represents the cDNA coding for the native human Abl protein (human c-abl mRNA; GenBank Accession No.: X16416).

SEQ ID NO:2 represents the amino acid sequence of the native human Abl protein (human c-Abl; SwissProt Acc. No.: P00519).

Unless indicated otherwise, the number given for a certain amino acid refers to the numbering of the amino acids in SEQ ID NO:2. In an amino acid sequence that is essentially similar to the amino acid sequence of the native human Abl kinase domain within the meaning as defined above, the amino acids are numbered in accordance with the numbering of the amino acids in SEQ ID NO:2.

The term "isolated" means that the material is removed from its original environment (e.g., the natural environment if it is naturally occurring).

A "host cell", refers to a prokaryotic or eukaryotic cell that contains heterologous DNA that has been introduced into the cell by any means, e.g., electroporation, calcium phosphate precipitation, microinjection, transformation, viral infection, and the like.

### Description of the Invention:

In practicing the present invention, many conventional techniques in molecular biology, microbiology, and recombinant DNA are used. These techniques are well known and are explained in, for example, Current Protocols in Molecular Biology, Volumes I, II, and III, 1997 (F. M. Ausubel ed.); Sambrook et al., 1989, Molecular Cloning: A Laboratory Manual, Second Edition, Cold Spring Harbor Laboratory Press, Cold Spring Harbor, N.Y.; DNA Cloning: A Practical Approach, Volumes I and II, 1985 (D. N. Glover ed.); Oligonucleotide Synthesis, 1984 (M. L. Gait ed.); Nucleic Acid Hybridization, 1985, (Hames and Higgins); Transcription and Translation, 1984 (Hames and Higgins eds.); Animal Cell Culture, 1986 (R. I. Freshney ed.); Immobilized Cells and Enzymes, 1986 (IRL Press); Perbal, 1984, A Practical Guide to Molecular Cloning; the series, Methods in Enzymology (Academic Press, Inc.); Gene Transfer Vectors for Mammalian Cells, 1987 (J. H. Miller and M. P. Calos eds., Cold Spring Harbor Laboratory); and Methods in Enzymology Vol. 154 and Vol. 155 (Wu and Grossman, and Wu, eds., respectively).

In particular, the polypeptides of the present invention can be produced by recombinant DNA technology using techniques well-known in the art. Methods which are well known to those skilled in the art can be used to construct expression vectors containing the sequences encoding the polypeptides of the invention and appropriate transcriptional/translational control signals. A variety of host-expression vector systems can be utilized to express the polypeptides of the invention.
(1) The invention relates to an isolated polypeptide which comprises a functional kinase domain comprising the amino acid sequence of the native human Abl kinase domain or an essentially similar sequence thereof in which Met244, is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamideora salt thereof.
(2) The invention relates to an isolated polypeptide which comprises a functional kinase domain comprising the amino acid sequence of the native human Abl kinase domain or an essentially similar sequence thereof in which Met244, is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof, and wherein optionally at least one additional amino acid selected from Gly250, Tyr253, Val256, Glu258, Ile313, Met318, Leu370, Phe382 and His396 is replaced by another amino acid.
(3) A preferred embodiment of the invention relates to an isolated polypeptide according to any one of the preceding paragraphs (1) - (2), wherein in the amino acid sequence of the native human Abl kinase domain or an essentially similar sequence thereof a single amino acid is replaced by another amino acid.
(4) In a second preferred embodiment the invention relates to an isolated polypeptide which comprises a functional kinase domain comprising the amino acid sequence of the native human Abl kinase domain or an essentially similar sequence thereof that contains Met244Val, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(5) In a preferred embodiment the invention relates to an isolated polypeptide according to any one of the preceding paragraphs (1)-(4), wherein the amino acid sequence of the native human Abl kinase domain consists of amino acids 229-500 of SEQ ID NO:2.
(6) In another preferred embodiment the invention relates to an isolated polypeptide according to any one of the preceding paragraphs (1)-(5); said isolated polypeptide being a Bcr-Abl tyrosine kinase.
(7) In yet another preferred embodiment the invention relates to the use of an isolated polypeptide of any one of the preceding paragraphs (1)-(6) to screen for compounds which inhibit the tyrosine kinase activity of said polypeptide.
(8) The invention also relates to an isolated nucleic acid molecule comprising a nucleotide sequence that encodes a polypeptide according to any one of the preceding paragraphs (1)-(6).
(9) The invention further relates to the use of a nucleic acid molecule of the preceding paragraph (8) in the production of a polypeptide of any one of the preceding paragraphs (1)-(6) for use in screening for compounds which inhibit the tyrosine kinase activity of said polypeptide.
(10) The invention also relates to a recombinant vector comprising a nucleic acid molecule according to the preceding paragraph (27).
(11) The invention further relates especially to a recombinant vector according to the preceding paragraph (10), which is a recombinant expression vector.
(12) The invention also relates to a host cell comprising a recombinant vector according to the preceding paragraph (10) or (11).

Preferably the invention relates to an isolated polypeptide which comprises a functional kinase domain comprising the amino acid sequence of the native human Abl kinase domain in which at least one amino acid is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.

A preferred salt of STI571 is the methane sulfonate salt described in WO 99/03854.

Screening for compounds which inhibit the tyrosine kinase activity of the polypeptides of the invention may be done for example by using an isolated polypeptide of the invention in any *in vitro* tyrosine kinase phosphorylation assay known in the art and determining the potential of a compound to inhibit the tyrosine kinase activity of a polypeptide of the invention in such an assay.
High-throughput screening assays known in the art may be used to screen large compound libraries for compounds which inhibit the tyrosine kinase activity of the polypeptides of the invention.
Besides the random screening of large compound libraries, the polypeptides of the present invention may also be used in the following screening approach: The 3-dimensional structure of a polypeptide of the invention is determined by e.g. X-ray crystallography. The atomic coordinates of a polypeptide of the invention are then used to design a potential inhibitor. Said potential inhibitor is then synthesized and tested for its ability to inhibit the tyrosine kinase activity of the polypeptide of the invention in any *in vitro* tyrosine kinase phosphorylation assay.

### Examples:

The following Examples serve to illustrate the invention without limiting its scope.

### Example 1:

### Example 4:

An RT-PCR strategy was used to amplify and sequence the Abl kinase domain of Bcr-Abl in 28 patients. We selected STI571-refractory and STI571-resistant patients from 253 patients enrolled in expanded access studies at 5 Australian centres. The aim was to determine the frequency and timing of acquired mutations within carefully defined clinical groups, determine their distribution within the Bcr-Abl kinase domain and identify any association between specific mutations and clinical features.

### Study Design

STI571-resistant patients (n=18) were defined as those with a loss of complete haematological remission which had been present for at least 3 months or evolution to acute phase CML or relapsed Ph+ ALL. STI571-refractory patients (n=10) were those who failed to achieve a major cytogenetic response after at least 6 months of therapy.

Extraction of RNA from blood, reverse transcription and DNA sequencing procedures have been previously described [Branford S. et al., Br. J. Haematol. 107, 587-599 (1999)]. A long PCR method [Branford S. et al., Br. J. Haematol. 109, 635-637 (2000)] was used to amplify the Abl kinase domain of Bcr-Abl with forward primer BcrF (5' tgaccaactcgtgtgtgaaactc) and reverse primer AbIKinaseR (5' tccacttcgtctgagatactggatt). A second stage PCR used forward primer AblkinaseF (5' cgcaacaagcccactgtct) and reverse primer AblkinaseR. The entire kinase domain was sequenced, an area including 863 bases (GenBank accession number M14752).

### Results

### STI571-resistant patients:

Twelve of 18 STI571-resistant patients had mutations in the ATP binding region of Bcr-Abl (Table 6). In 9 cases where samples were available, we confirmed that the mutation was not present before starting STI571 therapy, nor was it present in 4 cases tested at 3-9 months, which in each case was before the onset of resistance. Three of the 6 resistant patients without Bcr-Abl mutations had evidence of clonal evolution at the time of relapse including an additional Philadelphia (Ph) chromosome in 2 cases. One patient had both a mutation and an extra Ph chromosome at time of relapse.

The 6 mutations identified are T315I (n=3 patients), Y253H (n=1), F317L (n=1), E255K (n=4), G250E (n=2) and M351T (n=1).

The 18 STI571-resistant patients could be subdivided into those who relapsed into blast crisis or Ph+ ALL (n=8), those relapsing into accelerated phase (n=6) and those with evidence of loss of hematological remission who remained in chronic phase (n=4). All 3 groups included patients with mutations. Six of the 8 patients who relapsed directly into blast crisis/ALL had mutations. In 3 of these the T315I mutation was present. Two of the 6 patients relapsing into accelerated phase had mutations. The remaining 4 patients relapsing into chronic phase all had mutations.

### STI571-refractory patients:

Only 1 of 10 refractory patients had a mutation (Table 6). There was no evidence of the mutation pre-STI571 therapy or 3 months after starting treatment. The mutant clone mixed with wild-type Bcr-Abl emerged at 8 months and persisted in a mixed pattern until the mutant clone became predominant at 11 months. To date there has been no clinical evidence of resistance in this patient.

**Table 6. Clinical course and mutation analysis of patients treated with STI571**

| Patient ID | Age /Sex | Disease status at start of STI571* | Duration of STI571 Treatment | Best Response to STI571* | Response at Time of Mutation Analysis* | Time of Mutation Analysis † | Mutation Result | Nucleotide Substitution (GenBank no. M14752) |
|---|---|---|---|---|---|---|---|---|
| Emerging Resistance | | | | | | | | |
| 01 | 61 F | 3 (3^{rd}) | 9m | | 1b | Pre Study | NM | |
| | | | | 7 | 7 | 3m | NM | |
| | | | | | 7 | 6m | NM | |
| | | | | | 1b | 9m | T315I | G to C nt 944 |
| | | | | | | | | |
| 02 | 75 F | 1a | 4.5m | | 1a | Pre study | | |
| | | | | 4 | 1a | 4.5m | T315I | G to C nt 944 |
| | | | | | | | | |
| 03 | 62 M | 1b | 2m | | 1b | Pre study | | |
| | | | | 4 | 1b | 2m | T315I | G to C nt 944 |
| | | | | | | | | |
| 04§ | 59 F | 1c | 3m | | 1c | Pre Study | NM | |
| | | | | 7 | 1c | 5m | Y253H | T to C nt 757 |
| | | | | | 1c | 5.5m | Y253H | T to C nt 757 |
| 05 | 40 M | 3 | 8m | | 3 | Pre Study | NM | |
| | | | | 5 | 5 | 4m | F317L | C to G nt 951 |
| | | | | | 4 | 7m | F317L | C to G nt 951 |
| | | | | | | | | |
| 06 | 66 M | 1b | 8m‡ | 4 | 1b | 8m | G250E | G to C nt 749 |
| | | | | | | | | |
| 07 | 54 F | 1a | 10.5m | | 1a | Pre Study | NM | |
| | | | | 5 | 5 | 6m | NM | |
| | | | | | 5 | 9m | NM | |
| | | | | | 5 | 10m | G250E | G to C nt 749 |
| | | | | | 4 | 10.5m | G250E | G to C nt 749 |
| | | | | | | | | |
| 08 | 41 M | 2 | 6m | 5 | 2 | 6m | E255K | G to A nt 769 |
| | | | | | | | | |
| 09 | 60 M | 2 | 5m | 5 | 5 | 3m | NM | |
| | | | | | | | | |
| | | | | | 4 | 6m | E255K | G to A nt 769 |
| | | | | | | | | |
| 10 | 44 M | 2 | 4.5m | | 2 | Pre Study | NM | |
| | | | | 5 | 4 | 4m | E255K | G to A nt 769 |
| | | | | | | | | |
| 11§ | 60 F | 1c | 3m | | 1c | Pre Study | NM | |
| | | | | 5 | 1c | 3m | E255K | G to A nt 769 |
| | | | | | | | | |
| 12 | 52 F | 3 | 9m | | 3 | Pre Study | NM | |
| | | | | 7 | 7 | 6m | NM | |
| | | | | | 7 | 7m | NM | |
| | | | | | 6 | 8m | M351T | T to C nt 1052 |
| | | | | | 2 ^{P} | 9m | M351T | T to C nt 1052 |
| | | | | | | | | |
| 13 | 64 M | 3 | 10.5m | 5 | 2 ^{P} | 10m | NM | |
| | | | | | | | | |
| 14 | 78 F | 1a | 8m | 5 | 2 ^{C+P} | 8m | NM | |
| | | | | | | | | |
| 15 | 47 M | 2^{C} | 9m | 2 ^{C} | 1 ^{C} | 7m | NM | |
| | | | | | | | | |
| 16 | 56 F | 3 | 7m | 5 | 2 | 6m | NM | |
| | | | | | | | | |
| 17 | 37 M | 1a | 7m | 4 | 1a | 4.5m | NM | |
| | | | | | | | | |
| 18 | 63 M | 2 | 7m | 4 | 2 | 8m | NM | |
| Refractory | | | | | | | | |
| 19 | 55 F | 2 ^{C} | 6m | 4 | 4 ^{C} | 3.5m | NM | |
| | | | | | | | | |
| 20 | 63 M | 2 ^{C} | 11m | | 2 ^{C} | Pre Study | NM | |
| | | | | 2 ^{C} | 2 ^{C} | 3m | NM | |
| | | | | | 2 ^{C} | 8m | M351T | T to C nt 1052 |
| | | | | | 2 ^{C} | 9m | M351T | T to C nt 1052 |
| | | | | | 2 ^{C} | 10m | M351T | T to C nt 1052 |
| | | | | | 2 ^{C} | 11m | M351 T | T to C nt 1052 |
| | | | | | | | | |
| 21 | 62 F | 2 | 10.5m | 5 | 4 | 9m | NM | |
| | | | | | | | | |
| 22 | 54 M | 2 | 8m | 5 | 5 | 6m | NM | |
| | | | | | | | | |
| 23 | 61 F | 3 | 6m | 5 | 4 | 5m | NM | |
| | | | | | | | | |
| 24 | 61 F | 3 | 10.5m | 5 | 4 | 6m | NM | |
| | | | | | | | | |
| 25 | 47 F | 3 | 10m | 5 | 5 | 6m | NM | |
| | | | | | | | | |
| 26 | 40 F | 3 | 6m | 5 | 5 | 3m | NM | |
| | | | | | | | | |
| 27 | 60 M | 3 | 6m | 5 | 5 | 4m | NM | |
| | | | | | | | | |
| 28 | 42 M | 3 | 7m | 5 | 5 | 6m | NM | |

| | | | | | | | | |
|---|---|---|---|---|---|---|---|---|
| F: female; M: male; m: months. NM Indicates no mutation detected following sequencing of the kinase region of Bcr-Abl in both directions. ^{C}Indicates clonal evolution. ^{P}Indicates double Philadelphia chromosome. *Disease Status/Response: 1 a) myeloid blast crisis b) lymphoid blast crisis c) Ph-positive ALL relapse. 2) Accelerated Phase, 3) Chronic Phase, 4) Partial Response (blasts in blood + bone marrow < 15%), 5) Complete Haematologic Response (WBC<10.0, Pits < 450, no blasts, myelocytes + metamyelocytes < 5%, no promyelocytes, no disease-related symptoms or extramedullary disease), 6) Major Cytogenetic Response, 7) Complete Cytogenetic Response. †Months since treatment start. ‡Although patient was on study for 8 months, STI571 was alternated monthly with oral arsenic and therefore the patient received only 4 months of STI571 over this period. §Ph-positive ALL patients. | | | | | | | | |

Further studies revealed the presence of two additional mutations, i.e. Met244Val and Phe486Ser, in STI571-resistant/refractory patients suffering from Ph chromosome-positive leukemia.
In another study, CML patients from 12 centers within Australia and New Zealand were tested for mutation analysis. The samples were primarily collected for molecular assessment of BCR-ABL levels and only proceeded to mutation analysis if stored RNA contained a measurable level of BCR-ABL and the control gene level indicated non-degraded RNA. Patients had either received 6 or more months of imatinib therapy or had developed resistance and ceased therapy before 6 months (n=2). Samples from 156 patients were available but 12 could not be tested, 4 due to low levels of BCR-ABL and 8 due to inadequate RNA quality. The remaining 144 patients were grouped according to the disease stage at start of imatinib; 40 in AP, 64 in late-CP defined as ≥12 months since diagnosis and 40 in early-CP defined as <12 months since diagnosis. Sixteen of the early-CP patients had failed previous interferon therapy and 24 had only received hydroxyurea prior to imatinib therapy. Response to imatinib was categorised by the cytogenetic analysis at 6 months as either a major cytogenetic response (MCR) if the number of Philadelphia chromosome positive cells was <35% or no MCR if 35-100%. Imatinib resistance was defined as loss of a complete hematologic remission (CHR) that had been present for at least 3 months, loss of CHR with transformation to accelerated or blastic phase, or loss of an established MCR or a complete cytogenetic response (CCR defined as Philadelphia chromosome negative). 346 RNA samples were analysed as already mentioned herein. Depending on available RNA, patients were tested for mutations at between 1-15 different time-points. The median duration of imatinib therapy was therefore determined from the last time-point of analysis. AP patients had received a median of 9 months of imatinib (range 4 to 24), late-CP 10 months (range 6-24) and early-CP 14 months (range 5-24).

### Imatinib resistance in accelerated phase patients

Fourteen of 40 AP patients developed imatinib resistance, 5 with transformation to blast phase, 8 with recurrence of AP and 1 with loss of CCR. Mutations were detected in 12 of 14 (86%) resistant patients at a median of 8 months of imatinib therapy (range 4-13).

### Imatinib resistance in late chronic phase patients (late-CP)

Thirteen of 64 late-CP patients developed imatinib resistance, 5 with transformation to blast phase, 6 to AP and 2 lost a MCR. Mutations were detected in 11 of 13 (85%) resistant patients at a median of 8 months of imatinib therapy (range 3-18).

### Imatinib resistance in early chronic phase patients (early-CP)

Six of 40 early-CP patients developed resistance, 1 transformed to blast crisis, 2 to AP, 2 lost CCR and 1 lost MCR. No mutations were detected in the resistant patients.

### Duration of CML and the development of mutations

When all the patients in the study who achieved a major cytogenetic response (MCR) within 6 months were studied, those treated >4 years since diagnosis had a 9 times higher incidence of mutations than those treated within 4 years (6 of 22 (27%) versus 2 of 72 (3%)). When all the patients who did not achieve a MCR within 6 months were studied, those treated >4 years since diagnosis had a 2 times higher incidence of mutations than those treated within 4 years (12 of 22 (54%) versus 7 of 28 (25%)). The highest incidence of mutations was in AP patients treated >4 years from diagnosis and who failed to achieve a MCR by 6 months (8 of 12, 67%). In this study 27 of the 144 patients developed mutations. The median duration of CML prior to commencing imatinib therapy of the 27 patients with mutations (5.3 years, range 1.1 to 11) was statistically different to the 117 patients without mutations (1.3 years, range 0.02-17.7) p<0.0001.

### Mutations in the BCR-ABL kinase domain

Table 7 details the 17 different mutations in the BCR-ABL kinase domain detected in 27 patients. These were all point mutations and were located within a sequence of 728 nucleotides involving amino acids 244 to 486. Seven patients had 2-4 mutations and one patient had 2 different mutations at the same nucleotide which both altered the amino acid at position 252 from glutamine to histidine. The mutations, L248V at the N-terminal and S417Y, E459K and F486S at the C-terminal of the kinase domain have not previously been described. The first 3 mutations were all detected in one imatinib resistant patient who also had the G250E mutation.

**Table 7. BCR-ABL kinase domain mutations**

| Mutation | Nucleotide Substitution (GenBank number M14752) | Number of patients with the mutation* |
|---|---|---|
| M244V | 730A>G | 1 |
| L248V | 742C>G | 1 |
| G250E | 749G>A | 3 |
| Q252H | 756G>C | 3 |
| Q252H | 756G>T | 1 |
| Y253F | 758A>T | 2 |
| E255K | 763G>A | 1 |
| E255V | 764A>T | 5 |
| T315I | 944C>T | 2 |
| F317L | 951C>G | 2 |
| M351T | 1052T>C | 8 |
| E355G | 1064T>C | 3 |
| F359V | 1075T>G | 2 |
| H396R | 1187A>G | 1 |
| S417Y | 1250C>A | 1 |
| E459K | 1375G>A | 1 |
| F486S | 1457T>C | 1 |

| | | |
|---|---|---|
| *7 patients had multiple mutations | | |

## Claims

1. An isolated polypeptide which comprises a functional kinase domain comprising the amino acid sequence of the native human Abl kinase domain or an essentially similar sequence thereof in which Met244 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.

2. The isolated polypeptide according to claim 1, wherein Met244 is replaced by Valine.

3. The isolated polypeptide according to claim 1 or 2, wherein the amino acid sequence of the native human Abl kinase domain consists of amino acids 229-500 of SEQ ID NO:2.

4. The isolated polypeptide according to any one of claims 1 to 3, which is a Bcr-Abl tyrosine kinase.

5. Use of a polypeptide according to any one of claims 1 to 4 to screen for compounds which inhibit the tyrosine kinase activity of said polypeptide.

6. An isolated nucleic acid molecule comprising a nucleotide sequence that encodes a polypeptide according to any one of claims 1 to 4.

7. Use of a nucleic acid molecule according to claim 6 in the production of a polypeptide according to any one of claims 1 to 4 for use in screening for compounds which inhibit the tyrosine kinase activity of said polypeptide.

8. A recombinant vector comprising a nucleic acid molecule according to claim 6.

9. The recombinant vector according to claim 8, which is a recombinant expression vector.

10. A host cell comprising a recombinant vector according to claim 8 or 9.

## Patentansprüche

1. Isoliertes Polypeptid, das eine funktionale Kinasedomäne umfasst, umfassend die Aminosäuresequenz der nativen humanen Abl-Kinasedomäne oder eine im Wesentlichen ähnliche Sequenz hiervon, worin Met 244 durch eine andere Aminosäure ersetzt ist, wobei diese funktionale Kinasedomäne resistent ist gegen eine Inhibition ihrer Tyrosinkinaseaktivität durch N-[4-Methyl-3-(4-pyridin-3-yl-pyrimidin-2-ylamino)-phenyl]-4-(4-methylpiperazin-1-ylmethyl)-benzamid oder ein Salz hiervon.

2. Isoliertes Polypeptid nach Anspruch 1, worin Met 244 ersetzt ist durch Valin.

3. Isoliertes Polypeptid nach Anspruch 1 oder 2, worin die Aminosäuresequenz der nativen humanen Abl-Kinasedomäne aus den Aminosäuren 229-500 von SEQ ID NR:2 besteht.

4. Isoliertes Polypeptid nach einem der Ansprüche 1 bis 3, das eine Bcr-Abl-Tyrosinkinase ist.

5. Verwendung eines Polypeptids nach einem der Ansprüche 1 bis 4, zum Screenen nach Verbindungen, die die Tyrosinkinaseaktivität dieses Polypeptids inhibieren.

6. Isoliertes Nucleinsäuremolekül, umfassend eine Nucleotidsequenz, die für ein Polypeptid nach einem der Ansprüche 1 bis 4 kodiert.

7. Verwendung eines Nucleinsäuremoleküls nach Anspruch 6 zur Herstellung eines Polypeptids nach einem der Ansprüche 1 bis 4 zur Verwendung beim Screenen nach Verbindungen, die die Tyrosinkinaseaktivität dieses Polypeptids inhibieren.

8. Rekombinanter Vektor, umfassend ein Nucleinsäuremolekül nach Anspruch 6.

9. Rekombinanter Vektor nach Anspruch 8, der ein rekombinanter Expressionsvektor ist.

10. Wirtszelle, umfassend einen rekombinanten Vektor nach Anspruch 8 oder 9.

## Revendications

1. Polypeptide isolé comprenant un domaine kinase fonctionnel comprenant la séquence d'acides aminés du domaine kinase Ab1 humain natif, ou l'une de ses séquences essentiellement similaires, dans laquelle Met244 est remplacée par un autre acide aminé, ledit domaine kinase fonctionnel résistant à l'inhibition de son activité tyrosine kinase par le N-[4-méthyl-3-(4-pyridin-3-yl-pyrimidin-2-ylamino)-phényl]-4-(4-méthyl-pipérazin-1-ylméthyl)-benzamide ou un sel de celui-ci.

2. Polypeptide isolé selon la revendication 1, dans lequel l'acide aminé Met244 est remplacé par l'acide aminé valine.

3. Polypeptide isolé selon la revendication 1 ou 2, dans lequel la séquence d'acides aminés du domaine kinase Ab1 humain natif consiste en les acides aminés 229 à 500 de SEQ ID N°:2.

4. Polypeptide isolé selon l'une quelconque des revendications 1 à 3, qui est une tyrosine kinase Bcr-Abl.

5. Utilisation d'un polypeptide selon l'une quelconque des revendications 1 à 4, pour le criblage de composés qui inhibent l'activité tyrosine kinase dudit polypeptide.

6. Molécule d'acide nucléique isolée comprenant une séquence nucléotidique codant pour un polypeptide selon l'une quelconque des revendications 1 à 4.

7. Utilisation d'une molécule d'acide nucléique selon la revendication 6, dans la préparation d'un polypeptide selon l'une quelconque des revendications 1 à 4, pour le criblage de composés qui inhibent l'activité tyrosine kinase dudit polypeptide.

8. Vecteur recombinant comprenant une molécule d'acide nucléique selon la revendication 6.

9. Vecteur recombinant selon la revendication 8, qui est un vecteur d'expression recombinant.

10. Cellule hôte comprenant un vecteur recombinant selon la revendication 8 ou 9.


### SEQUENCE LISTING

<110> Novartis AG
   <110> Université Victor Segalen Bordeaux 2
   <110> Oregon Health & Science University
   <110> University of Heidelberg
   <110> Le Centre Hospitalier Régional Universitaire de Lille
   <110> Medvet Science Pty Ltd
   <110> Technische Universität München
<120> Mutated Abl kinase domains
   <130> Case 4-32176A
<150> US 60/327389
   <151> 2001-10-5
<150> US 60/328740
   <151> 2001-10-12
<150> US 60/347351
   <151> 2002-01-11
<160> 2
<170> PatentIn version 3.0
<210> 1
   <211> 3393
   <212> DNA
   <213> Homo sapiens
<220>
   <221> CDS
   <222> (1)..(3393)
<400> 1
   atg ttg gag atc tgc ctg aag ctg gtg ggc tgc aaa tcc aag aag ggg        48
   ctg tcc tcg tcc tcc agc tgt tat ctg gaa gaa gcc ctt cag cgg cca        96
   gta gca tct gac ttt gag cct cag ggt ctg agt gaa gcc gct cgt tgg      144
   aac tcc aag gaa aac ctt ctc gct gga ccc agt gaa aat gac ccc aac      192
   ctt ttc gtt gca ctg tat gat ttt gtg gcc agt gga gat aac act cta      240
   agc ata act aaa ggt gaa aag ctc cgg gtc tta ggc tat aat cac aat      288
   ggg gaa tgg tgt gaa gcc caa acc aaa aat ggc caa ggc tgg gtc cca      336
   agc aac tac atc acg cca gtc aac agt ctg gag aaa cac tcc tgg tac      384
   cat ggg cct gtg tcc cgc aat gcc gct gag tat ctg ctg agc agc ggg      432
   atc aat ggc agc ttc ttg gtg cgt gag agt gag agc agt cct ggc cag      480
   agg tcc atc tcg ctg aga tac gaa ggg agg gtg tac cat tac agg atc      528
   aac act gct tct gat ggc aag ctc tac gtc tcc tcc gag agc cgc ttc      576
   aac acc ctg gcc gag ttg gtt cat cat cat tca acg gtg gcc gac ggg 624
   ctc atc acc acg ctc cat tat cca gcc cca aag cgc aac aag ccc act 672
   gtc tat ggt gtg tcc ccc aac tac gac aag tgg gag atg gaa cgc acg 720
   gac atc acc atg aag cac aag ctg ggc ggg ggc cag tac ggg gag gtg 768
   tac gag ggc gtg tgg aag aaa tac agc ctg acg gtg gcc gtg aag acc 816
   ttg aag gag gac acc atg gag gtg gaa gag ttc ttg aaa gaa gct gca 864
   gtc atg aaa gag atc aaa cac cct aac ctg gtg cag ctc ctt ggg gtc 912
   tgc acc cgg gag ccc ccg ttc tat atc atc act gag ttc atg acc tac 960
   ggg aac ctc ctg gac tac ctg agg gag tgc aac cgg cag gag gtg aac 1008
   gcc gtg gtg ctg ctg tac atg gcc act cag atc tcg tca gcc atg gag 1056
   tac ctg gag aag aaa aac ttc atc cac aga gat ctt gct gcc cga aac      1104
   tgc ctg gta ggg gag aac cac ttg gtg aag gta gct gat ttt ggc ctg      1152
   agc agg ttg atg aca ggg gac acc tac aca gcc cat gct gga gcc aag      1200
   ttc ccc atc aaa tgg act gca ccc gag agc ctg gcc tac aac aag ttc      1248
   tcc atc aag tcc gac gtc tgg gca ttt gga gta ttg ctt tgg gaa att      1296
   gct acc tat ggc atg tcc cct tac ccg gga att gac ctg tcc cag gtg      1344
   tat gag ctg cta gag aag gac tac cgc atg gag cgc cca gaa ggc tgc      1392
   cca gag aag gtc tat gaa ctc atg cga gca tgt tgg cag tgg aat ccc      1440
   tct gac cgg ccc tcc ttt gct gaa atc cac caa gcc ttt gaa aca atg      1488
   ttc cag gaa tcc agt atc tca gac gaa gtg gaa aag gag ctg ggg aaa      1536
   caa ggc gtc cgt ggg gct gtg agt acc ttg ctg cag gcc cca gag ctg      1584
   ccc acc aag acg agg acc tcc agg aga gct gca gag cac aga gac acc 1632
   act gac gtg cct gag atg cct cac tcc aag ggc cag gga gag agc gat 1680
   cct ctg gac cat gag cct gcc gtg tct cca ttg ctc cct cga aaa gag 1728
   cga ggt ccc ccg gag ggc ggc ctg aat gaa gat gag cgc ctt ctc ccc 1776
   aaa gac aaa aag acc aac ttg ttc agc gcc ttg atc aag aag aag aag 1824
   aag aca gcc cca acc cct ccc aaa cgc agc agc tcc ttc cgg gag atg 1872
   gac ggc cag ccg gag cgc aga ggg gcc ggc gag gaa gag ggc cga gac 1920
   atc agc aac ggg gca ctg gct ttc acc ccc ttg gac aca gct gac cca 1968
   gcc aag tcc cca aag ccc agc aat ggg gct ggg gtc ccc aat gga gcc 2016
   ctc cgg gag tcc ggg ggc tca ggc ttc cgg tct ccc cac ctg tgg aag 2064
   aag tcc agc acg ctg acc agc agc cgc cta gcc acc ggc gag gag gag      2112
   ggc ggt ggc agc tcc agc aag cgc ttc ctg cgc tct tgc tcc gcc tcc      2160
   tgc gtt ccc cat ggg gcc aag gac acg gag tgg agg tca gtc acg ctg      2208
   cct cgg gac ttg cag tcc acg gga aga cag ttt gac tcg tcc aca ttt      2256
   gga ggg cac aaa agt gag aag ccg gct ctg cct cgg aag agg gca ggg      2304
   gag aac agg tct gac cag gtg acc cga ggc aca gta acg cct ccc ccc      2352
   agg ctg gtg aaa aag aat gag gaa gct gct gat gag gtc ttc aaa gac      2400
   atc atg gag tcc agc ccg ggc tcc agc ccg ccc aac ctg act cca aaa      2448
   ccc ctc cgg cgg cag gtc acc gtg gcc cct gcc tcg ggc ctc ccc cac      2496
   aag gaa gaa gct gaa aag ggc agt gcc tta ggg acc cct gct gca gct      2544
   gag cca gtg acc ccc acc agc aaa gca ggc tca ggt gca cca ggg ggc      2592
   acc agc aag ggc ccc gcc gag gag tcc aga gtg agg agg cac aag cac      2640
   tcc tct gag tcg cca ggg agg gac aag ggg aaa ttg tcc agg ctc aaa      2688
   cct gcc ccg ccg ccc cca cca gca gcc tct gca ggg aag gct gga gga      2736
   aag ccc tcg cag agc ccg agc cag gag gcg gcc ggg gag gca gtc ctg      2784
   ggc gca aag aca aaa gcc acg agt ctg gtt gat gct gtg aac agt gac      2832
   gct gcc aag ccc agc cag ccg gga gag ggc ctc aaa aag ccc gtg ctc      2880
   ccg gcc act cca aag cca cag tcc gcc aag ccg tcg ggg acc ccc atc      2928
   agc cca gcc ccc gtt ccc tcc acg ttg cca tca gca tcc tcg gcc ctg      2976
   gca ggg gac cag ccg tct tcc act gcc ttc atc cct ctc ata tca acc      3024
   cga gtg tct ctt cgg aaa acc cgc cag cct cca gag cgg atc gcc          3069
   agc ggc gcc atc acc aag ggc gtg gtc ctg gac agc acc gag gcg      3114
   ctg tgc ctc gcc atc tct agg aac tcc gag cag atg gcc agc cac      3159
   agc gca gtg ctg gag gcc ggc aaa aac ctc tac acg ttc tgc gtg      3204
   agc tat gtg gat tcc atc cag caa atg agg aac aag ttt gcc ttc      3249
   cga gag gcc atc aac aaa ctg gag aat aat ctc cgg gag ctt cag      3294
   atc tgc ccg gcg aca gca ggc agt ggt ccg gcg gcc act cag gac      3339
   ttc agc aag ctc ctc agt tcg gtg aag gaa atc agt gac ata gtg      3384
   cag agg tag                                                      3393
<210> 2
   <211> 1130
   <212> PRT
   <213> Homo sapiens
<400> 2